# EUROPEAN PATENT APPLICATION

(11) **EP 4 144 830 A1**
(43) Date of publication of application: **08.03.2023**
(21) Application number: 21797892.3
(22) Date of filing: 05.04.2021
(51) Int. Cl.: C12N 1/00, C12N 5/0735, C12N 5/074, C12N 5/0775, C12N 5/0789, C12N 5/0797, C12N 5/10

(54) **STEM CELL MEDIUM AND STEM CELL CULTURING METHOD**

(30) Priority: 30.04.2020 JP 2020080760
(71) Applicant: Oriental Yeast Co., Ltd., Tokyo 174-8505 (JP)
(72) Inventor: GA, Ayumi, Nagahama-shi, Shiga 526-0804 (JP); TOMIMORI, Yoshiya, Nagahama-shi, Shiga 526-0804 (JP); YASUDA, Hisataka, Nagahama-shi, Shiga 526-0804 (JP)
(74) Representative: Forstmeyer, Dietmar
(86) International application number: PCT/JP2021/014552
(87) International publication number: WO 2021/220731

(57) **Abstract**

A medium for stem cells according to the present invention contains at least one of carboxymethyl cellulose and polyvinylpyrrolidone as a water-soluble polymer. The content of carboxymethyl cellulose in the medium is preferably such that the final concentration thereof is 0.001 µg/mL to 1 mg/mL. The content of polyvinylpyrrolidone in the medium is preferably such that the final concentration thereof is 0.05 µg/mL to 2 mg/mL.

## Description

### Technical Field

The present invention relates to a medium for stem cells and a method for culturing stem cells.

### Background Art

Stem cells as typified by multipotent stem cells are undifferentiated cells that have a self-renewal ability and can be differentiated into various types of cells. In recent years, regenerative medicine is intensively studied, in which multipotent stem cells or cells obtained by inducing differentiation of multipotent stem cells are transplanted into impaired tissue of a patient to restore the impaired functions thereof. In the regenerative medicine, it is necessary to prepare a large amount of stem cells such as multipotent stem cells or mesenchymal stem cells or a large amount of differentiated cells obtained therefrom. Accordingly, studies are conducted on methods for efficiently proliferating the stem cells and methods for efficiently differentiating the stem cells.

To culture stem cells, additives such as various types of cytokines, serum, and substitutes thereof (for example, albumin) are usually used in a basal medium for stem cells such as a mTeSR1 medium (Non-Patent Literature 2), an Essential-8 medium (Non-Patent Literature 3), or a Dulbecco's modified Eagle medium (DMEM) / F 12 medium.

Patent Literature 1 discloses a medium including reduced amounts of seven non-essential amino acids used as the additives. Patent Literature 2 discloses that albumin and polyvinyl alcohol are added to a medium. Patent Literature 3 discloses that a serum-free medium that contains a phospholipid and a fatty acid is used together with a plurality of cytokines to culture mesenchymal stem cells of stem cells.

### Citation List

### Patent Literatures

Patent Literature 1: US2017/0198258A1
Patent Literature 2: US2017/0009200A1
Patent Literature 3: US2012/0329087A1

### Non-Patent Literatures

Non-Patent Literature 1: Ludwig, et al., Nat. Methods, 2006, vol. 3 (8), pp. 637 - 46
Non-Patent Literature 2: Chen, et al., Nat. Methods, 2011, vol. 8 (5), pp. 424 - 9

### Summary of Invention

However, the techniques disclosed in Patent Literatures 1 to 3 and Non-Patent Literatures 1 and 2 do not provide sufficient effects including proliferation performance for stem cells, and thus further development of improved techniques has been required. Accordingly, it is an object of the present invention to provide a medium for stem cells that exhibits favorable proliferation performance while maintaining the differentiative ability of the stem cells, and a method for culturing stem cells that uses the medium for stem cells.

The inventors of the present application have conducted in-depth studies to solve the problem described above. As a result, they have found that when at least one water-soluble polymer of carboxymethyl cellulose and polyvinylpyrrolidone is incorporated into a medium, the resulting medium exhibits a favorable proliferation performance while maintaining the differentiative ability of stem cells, and have thus accomplished the present invention.

The present invention has been made based on the findings described above, and provides a medium for stem cells described below and also a method for culturing stem cells, the method including using the medium for stem cells.
[1] A medium for stem cells, comprising at least one of carboxymethyl cellulose and polyvinylpyrrolidone as a water-soluble polymer.
[2] The medium for stem cells as set forth in clause [1], wherein a content of carboxymethyl cellulose in the medium is such that a final concentration thereof is 0.001 µg/mL to 1 mg/mL.
[3] The medium for stem cells as set forth in clause [1], wherein a content of polyvinylpyrrolidone in the medium is such that a final concentration thereof is 0.05 µg/mL to 2 mg/mL.
[4] The medium for stem cells as set forth in any one of clauses [1] to [3], wherein a content of recombinant albumin in the medium is such that a final concentration thereof is 0.05 to 1 mg/mL.
[5] The medium for stem cells as set forth in any one of clauses [1] to [4], further containing β-nicotinamide mononucleotide.
[6] A method for culturing stem cells, comprising culturing stem cells in the medium for stem cells as set forth in any one of clauses [1] to [5].

### Brief Description of Drawings

[Fig. 1] Fig. 1 is a graph showing the results of measurement of absorbance of the media for stem cells obtained in Examples 1 to 7 and Comparative Example 1 using a crystal violet staining method in Proliferation Performance Test I.
[Fig. 2] Fig. 2 is a graph showing the results of measurement of absorbance of the media for stem cells obtained in Example 11 and Comparative Example 1 using a crystal violet staining method in Proliferation Performance Test I.
[Fig. 3] Fig. 3(a) is a graph showing the change in the cumulative number of divisions in the media for stem cells obtained in Example 8 and Comparative Example 1 in Proliferation Performance Test II, and Fig. 3(b) is a graph showing the change in the cumulative number of divisions in the media for stem cells obtained in Example 13 and Comparative Example 1 in Proliferation Performance Test II.
[Fig. 4] Fig. 4 is a graph showing the results of flow cytometry of the media for stem cells obtained in Example 8, Example 13, and Comparative Example 1 in Differentiative ability Test I.
[Fig. 5] Fig. 5 shows images showing the state of stem cells that have undergone induction of differentiation in Differentiative ability Test II in the media for stem cells obtained in Example 8, Example 13, and Comparative Example 1.

### Description of Embodiments

Hereinafter, the present invention will be described by way of preferred embodiments (modes) thereof.

A medium for stem cells according to the present invention is used to culture stem cells. The term "stem cells" refers to undifferentiated cells that have a self-renewal ability and an ability to be differentiated into various types of cells. The stem cells may be, for example, pluripotent stem cells, such as ES cells (embryonic stem cells) and iPS cells (induced pluripotent stem cells), and somatic stem cells, such as mesenchymal stem cells, hematopoietic stem cells, neural stem cells, and skin stem cells.

The stem cells to be cultured in the medium for stem cells according to the present invention are preferably animal-derived stem cells, more preferably mammal-derived stem cells, and even more preferably human-derived stem cells.

In view of obtaining more favorable proliferation performance while maintaining the differentiative ability, the stem cells to be cultured in the medium for stem cells according to the present invention are preferably stem cells that can be differentiated into any one of ectodermal cells, mesodermal cells, and endodermal cells, and more preferably stem cells that can be differentiated into mesodermal cells. Examples of such stem cells include mesenchymal stem cells.

The medium for stem cells according to the present invention contains at least one of carboxymethyl cellulose and polyvinylpyrrolidone as a water-soluble polymer. When incorporating any of these water-soluble polymers into a medium for stem cells, the resulting medium exhibits a favorable proliferation performance while maintaining the differentiative ability of stem cells.

Carboxymethyl cellulose (hereinafter also referred to as "CMC", CAS No. 9000-11-7) is a cellulose derivative, and specifically, cellulose ether obtained by substituting the hydroxy group(s) of glucopyranose of cellulose with a carboxymethyl group.

In the present invention, CMC conventionally used in media for culturing cells can be used without any particular limitation. A carboxymethyl cellulose salt may also be used.

In view of further improving the proliferation performance, CMC preferably has a degree of substitution with carboxymethyl ether groups per anhydroglucose unit (degree of etherification) of 0.6 to 0.8. Such CMC is commercially available. For example, commercial products such as carboxymethyl cellulose sodium salt (cat. no. 07326-95) available from Nacalai Tesque, Inc., and carboxymethyl cellulose (cat. no. 11676-85) available from Fuji Film Wako Pure Chemical Industries, Ltd. can be used.

Polyvinylpyrrolidone (hereinafter also referred to as "PVP", CAS No. 9003-39-8) is a water-soluble polymer obtained by polymerization of N-vinyl-2-pyrrolidone.

In the present invention, PVP conventionally used in media for culturing cells can be used without any particular limitation. In view of further improving the proliferation performance, PVP preferably has a weight average molecular weight of 40,000 to 1,200,000. Such PVP is commercially available. For example, commercial products such as polyvinylpyrrolidone K-30 (cat. no. 06306-72) available from Nacalai Tesque, Inc., and polyvinylpyrrolidone K-90 (cat. no. 28315-72) available from Nacalai Tesque, Inc. can be used.

At least one of CMC and PVP is contained in the medium for stem cells according to the present invention. Either one of or both of CMC and PVP may be contained in the medium for stem cells.

In view of further improving the proliferation performance while maintaining the differentiative ability of stem cells, the content of carboxymethyl cellulose in the medium for stem cells according to the present invention is such that the final concentration of carboxymethyl cellulose is preferably 0.001 µg/mL to 1 mg/mL, and more preferably 0.02 µg/mL to 0.5 mg/mL.

From the same viewpoint, the content of polyvinylpyrrolidone in the medium for stem cells according to the present invention is such that the final concentration of polyvinylpyrrolidone is preferably 0.05 µg/mL to 2 mg/mL, and more preferably 0.1 µg/mL to 1 mg/mL.

A medium used to culture stem cells usually contains an albumin (albumin protein) such as serum albumin.

In view of further improving the proliferation performance, the medium for stem cells according to the present invention preferably contains an albumin, more preferably contains an albumin from a mammal, and even more preferably contains an albumin from the same species as the stem cells to be cultured. The albumin is preferably a recombinant albumin. For example, a human-derived recombinant albumin is preferably used for culturing human-derived stem cells.

In the medium for stem cells according to the present invention, the amount of albumin, which is costly, can be advantageously reduced since at least one of carboxymethyl cellulose and polyvinylpyrrolidone is contained in the medium as described above. Accordingly, the content of albumin in the medium for stem cells according to the present invention can be such that the final concentration of albumin is 0.05 to 1 mg/mL, preferably 0.05 to 1 mg/mL, and more preferably 0.1 to 0.5 mg/mL.

In view of further improving the proliferation performance, it is preferable to use an albumin that carries a reduced amount of fatty acids. As such an albumin, an albumin that has undergone treatment for removing fatty acid, such as treatment with activated carbon, can be used as appropriate. The treatment for removing fatty acid can be performed using, for example, the method disclosed in WO 2014-192938.

In view of further improving the proliferation performance while maintaining the differentiative ability of stem cells, the medium for stem cells according to the present invention preferably contains β-nicotinamide mononucleotide. Regarding nicotinamide mononucleotide (hereinafter also referred to as "NMN", chemical formula: C₁₁H₁₅N₂O₈P), two types of optical isomers, α-form and β-form, are present, and it is preferable to use β-NMN (CAS No. 1094-61-7) in the medium for stem cells according to the present invention. The structure of β-NMN is shown below.

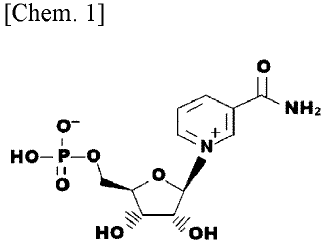

β-NMN prepared using any method can be used. For example, β-NMN artificially synthesized and purified using a chemical synthesis method, an enzyme method, or a fermentation method can be used as an active component. As the chemical synthesis method for synthesizing β-NMN, for example, a method can be used that includes allowing NAM and L-ribose tetraacetate to react with each other and phosphorylating the resulting nicotinamide mononucleotide to thereby obtain β-NMN. As the enzyme method, for example, a method can be used in which β-NMN is produced from NAM and 5'-phosphoribosyl-1'-pyrophosphate (PRPP) using nicotinamide phosphoribosyltransferase (NAMPT). As the fermentation method, for example, a method can be used in which β-NMN is produced from NAM by utilizing the metabolic system of a microorganism that expresses NAMPT.

β-NMN is a component present in a wide variety of organisms, and thus β-NMN obtained through extraction and purification from natural materials such as animals, plants, and microorganisms may also be used as an active component. Alternatively, commercially available purified β-NMN may also be used.

In view of further improving the proliferation performance while maintaining the differentiative ability of stem cells, the content of β-NMN in the medium for stem cells according to the present invention is such that the final concentration of β-NMN is preferably 0.01 to 5 mM, more preferably 0.05 to 2 mM, and even more preferably 0.1 to 1 mM.

As a basal medium of the medium for stem cells according to the present invention, an ordinary basal medium can be used, including a medium used to maintain the pluripotency (undifferentiated state) of stem cells or to proliferate stem cells or a medium used to culture animal cells. As the basal medium, it is also possible to use any of various types of commercially available culture media for stem cells. Examples of the basal medium include an Eagle's minimal essential medium (MEM), a Dulbecco's modified Eagle medium (DMEM), an α-Eagle's minimal essential medium (α-MEM), an Iscove's modified Dulbecco's medium (IMDM), an F-12 medium, an F-10 medium, a DMEM / F12 medium, a RPMI-1640 medium, a mesenchymal cell basal medium (MSCBM), an E8 (Essential 8) medium, a TeSR-E8 medium, an mTeSR1 medium, and mixed media thereof.

The medium for stem cells according to the present invention may optionally contain active components, such as amino acids, inorganic salts, vitamins, and antibiotics, in addition to the components described above. These active components may be used singly or in a combination of two or more. The active components that can be contained in the medium for stem cells according to the present invention may be components that are known to enhance the survival efficiency and proliferation efficiency of stem cells, and components that are known to enhance the differentiation efficiency, for example. The active components can be selected as appropriate from the following, for example: various types of amino acids; vitamins such as ascorbic acid and α-tocopherol; growth factors such as insulin and transferrin; minerals such as sodium selenite; bioactive substances such as ethanol amine, Rock inhibitor, valproic acid, dimethyl sulfoxide, dexamethasone, butyric acid, tricho statin A, GSK3 inhibitor, BMP inhibitor, and Wnt inhibitor; and cytokines such as PDGF-BB, EGF, VEGF, TGF-β, FGF2, activin, and noggin.

The active components described above are known to enhance the survival efficiency and proliferation efficiency of stem cells, or the action of maintaining the undifferentiated state of stem cells.

The method for culturing stem cells according to the present invention is a culturing method for efficiently proliferating stem cells while maintaining the differentiative ability of the stem cells. In other words, the method for culturing stem cells according to the present invention is a method for stably and efficiently culturing stem cells.

In the method for culturing stem cells according to the present invention, the medium for stem cells according to the present invention is suitably used. The method for culturing stem cells can be performed in the same manner as an ordinary method for culturing stem cells, except that at least one of carboxymethyl cellulose and polyvinylpyrrolidone is contained as a water-soluble polymer.

The conditions for culturing may be ordinary conditions in which animal cells can be cultured, and may be modified as appropriate where necessary. For example, stem cells can be cultured at a culture temperature of 30°C to 40°C, a CO₂ concentration of 1 to 10 vol%, and an O₂ concentration of 0.1 to 25 vol%.

### Examples

Next, the present invention will be described in further detail by way of examples. However, the present invention is not limited to the examples given below.

### Production of Medium for Stem Cells

A medium for stem cells was prepared. The medium had the same composition as that of serum-free medium A disclosed in Japanese Patent No. 5804385, except that CMC or PVP as a water-soluble compound was added instead of polyvinyl alcohol (PVA). The serum-free medium A was obtained by adding active components, such as FGF, PDGF, TGF-β, HGF, EGF, phospholipid, fatty acid, and PVA, to a basal medium obtained by mixing DEME (available from Sigma Co., cat. no. D6046) and MCDB 201 (available from Sigma Co., cat. no. M6770) at a ratio of 1:1 (see Table 1 in Japanese Patent No. 5804385). The content of albumin in the serum-free medium A was such that the final concentration thereof was 0.2 mg/mL. More specifically, media obtained by replacing PVA contained in the serum-free medium A by CMC (available from Nacalai Tesque, Inc., carboxymethyl cellulose sodium salt with a degree of etherification of 0.59 to 0.85, cat. no. 07326-95) were used as media for stem cells of Examples 1 to 10, and media obtained by replacing PVA contained in the serum-free medium A by PVP (available from Nacalai Tesque, Inc., polyvinylpyrrolidone K-90 with a weight average molecular weight of 360,000, cat. no. 28315-72) were used as media for stem cells of Examples 11 to 14. Also, β-NMN was further added to the media for stem cells of Examples 8 to 10 and Examples 12 to 14. A medium consisting of the serum-free medium A was used as a medium of Comparative Example 1.

The contents of CMC, PVP, and β-NMN in the media for stem cells (the final concentrations in the media) obtained in Examples and Comparative Examples are shown in the following table.

**[Table 1]**

| | Content of CMC in medium (final concentration) | Content of PVP in medium (final concentration) | Content of β-NMN in medium (final concentration) |
|---|---|---|---|
| Example 1 | 100 µg/mL | - | - |
| Example 2 | 20 µg/mL | - | - |
| Example 3 | 4 µg/mL | - | - |
| Example 4 | 0.8 µg/mL | - | - |
| Example 5 | 0.16 µg/mL | - | - |
| Example 6 | 0.032 µg/mL | - | - |
| Example 7 | 0.00128 ug/mL | - | - |
| Example 8 | 0.1 mg/mL | - | 0.25 mM |
| Example 9 | 0.2 mg/mL | - | 0.25 mM |
| Example 10 | 0.5 mg/mL | - | 0.25 mM |
| Example 11 | - | 0.128 µg/mL | - |
| Example 12 | - | 0.2 mg/mL | 0.25 mM |
| Example 13 | - | 0.4 mg/mL | 0.25 mM |
| Example 14 | - | 0.6 mg/mL | 0.25 mM |
| Comparative Example 1 | | | |

### Proliferation Performance Test I

A 96-well plate for culturing cells was coated with fibronectin (available from Corning Inc., product number: 356008) at 2.5 µg/cm². Stem cells were seeded in the 96-well plate at 800 cells/well with any one of the media for stem cells of Examples 1 to 7 and Comparative Example 1 in a scale of 100 µL, and the stem cells were statically cultured under conditions of 37°C and 5% CO₂. As the stem cells, human adipose-derived mesenchymal stem cells (available from Lonza Biologics, Inc., product number: PT-5006, 1 doner) were used. One day and four days after the seeding, the medium was exchanged, and the stem cells were cultured continuously for six days from the start of culturing. Here, the CMC-containing media for stem cells (the media for stem cells of Examples 1 to 7 and Comparative Example 1) described above were used for the culturing.

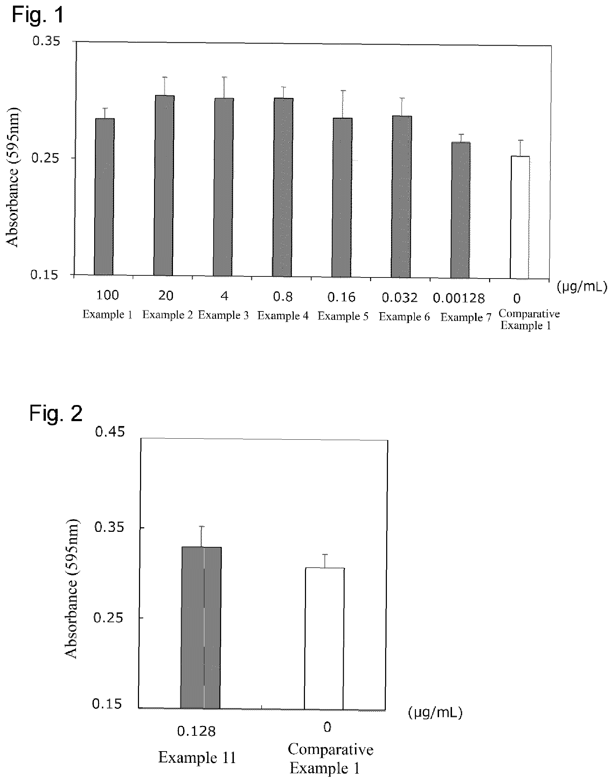

Next, proliferation performance for the stem cells was evaluated using a crystal violet staining method. First, the culture supernatant was removed from the 96-well plate, 0.99% paraformaldehyde (PFA) was added to each well, and the resultant was left to stand at 4°C for 1 hour or more. After that, PFA was removed from each well, and the well was washed with ultrapure water and 100% ethanol. As the ultrapure water, Milli-Q water was used. Milli-Q water is ultrapure water produced using an ultrapure water production system Milli-Q available from Millipore. After each well was washed, 2.5% crystal violet was added to the well, and the resultant was left to stand at room temperature for 10 minutes. Next, each well was further washed with tap water three times, and then dried. Then, absorbance at a wavelength of 595 nm was measured using a microplate reader. The measurement was performed on six independent wells, and the significance thereof was analyzed by performing a T-test. The measurement results are shown in Fig. 1.

Also, in a culture dish (60-mm dish) coated with fibronectin at 2.5 µg/cm², stem cells were seeded at 5 × 10³ cells/cm² with the medium for stem cells of Example 11 or Comparative Example 1 in a scale of 3 mL. Except for this, stem cells were cultured and the proliferation performance for the stem cells was evaluated in the same manner as in Examples 1 to 7 described above. The measurement results are shown in Fig. 2.

### Proliferation Performance Test II

In a culture dish (60-mm dish) coated with fibronectin at 2.5 µg/cm², the same commercially available stem cells as those used in Proliferation Performance Test I were seeded at 5 × 10³ cells/cm² with any one of the media for stem cells of Examples 8 to 10 and Comparative Example 1 in a scale of 3 mL, and the stem cells were statically cultured under conditions of 37°C and 5% CO₂. One day after the seeding, the medium was exchanged. After that, the medium for stem cells was exchanged once every two or three days. Cell passage was performed when the cells in the culture dish were grown to subconfluency (confluency of about 80 to 90%). More specifically, the culture supernatant was removed from the culture dish, and the dish was washed with phosphate-buffered saline (1 × PBS (-)). After that, 1 × Tryple select (available from Thermo Fisher, product number: 12563011) was added, and the resultant was left to stand under conditions of 37°C and 5% CO₂ for 4 minutes. Next, the cells were suspended by pipetting, and the cells removed from the culture dish were singulated. Next, the medium for stem cells was added, and centrifugation (1500 rpm, 5 min) was performed to remove the supernatant. The medium was added to the precipitated cells to suspend the cells therein. The number of cells in the suspension was counted, and the cells were seeded at 5 × 10³ cells/cm² in a culture dish (60-mm dish) coated with fibronectin in advance. In the culture dish after performing cell passage in this manner, the medium for stem cells was exchanged at a frequency of once every two or three days. The above-described cell passage was repeated eight times.

Next, the population doubling level was determined for the stem cells obtained through the cell passages described above and plotted in a graph. The population doubling level was calculated by adding the numbers of divisions of stem cells based on the number of initially seeded stem cells and the number of cells counted for each cell passage. Then, the obtained population doubling level was compared with the population doubling level obtained in Comparative Example 1 to evaluate the proliferation performance for stem cells of the medium of each Example on the following evaluation criteria. In the evaluation criteria, a medium in which the population doubling level was greater than that of Comparative Example 1 by one or more was considered that the medium exhibited "proliferation performance higher than that of Comparative Example 1", and a medium in which population doubling level was equal to that of Comparative Example 1 or less than that of Comparative Example 1 by one (minus one) was considered that the medium exhibited "proliferation performance equivalent to that of Comparative Example 1".
+: Proliferation performance was equivalent to that of Comparative Example 1.
++: Proliferation performance was higher than that of Comparative Example 1.

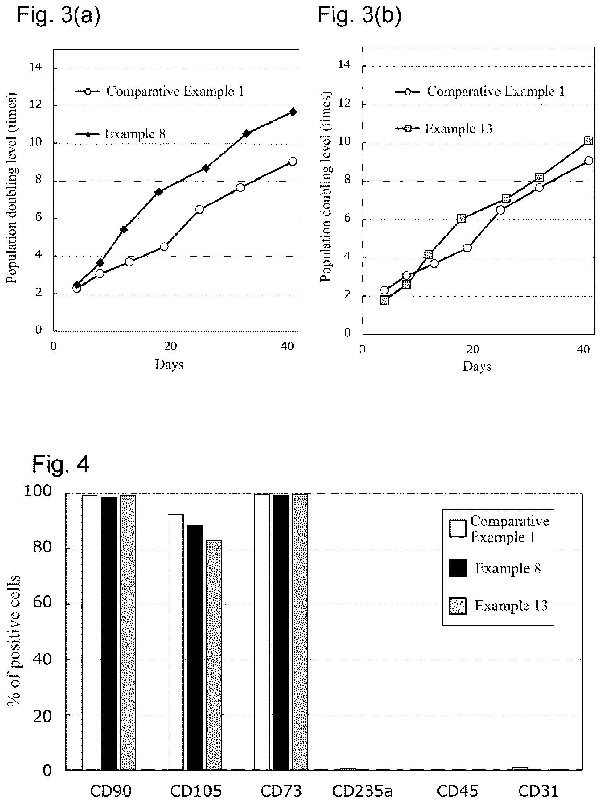

The evaluation results of Proliferation Performance Test II performed on the media for stem cells of Examples 8 to 10 and Comparative Example 1 are shown in Table 2. Also, the determination results of the population doubling level of the stem cells for Example 8 and Comparative Example 1 are shown in Fig. 3(a).

Proliferation Performance Test II described above was also performed on the media for stem cells of Examples 12 to 14 and Comparative Example 1. The evaluation results are shown in Table 3. Also, the determination results of the population doubling level of the stem cells for Example 13 and Comparative Example 1 are shown in Fig. 3(b).

**[Table 2]**

| | Comparative Example 1 | Example 8 | Example 9 | Example 10 |
|---|---|---|---|---|
| Content of CMC in medium (final concentration) | - | 0.1 mg/mL | 0.2 mg/mL | 0.5 mg/mL |
| Content of β-NMN in medium (final concentration) | - | 0.25 mM | 0.25 mM | 0.25 mM |
| Proliferation performance | + | ++ | ++ | ++ |

**[Table 3]**

| | Comparative Example 1 | Example 12 | Example 13 | Example 14 |
|---|---|---|---|---|
| Content of PVP in medium (final concentration) | - | 0.2 mg/mL | 0.4 mg/mL | 0.6 mg/mL |
| Content of β-NMN in medium (final concentration) | - | 0.25 mM | 0.25 mM | 0.25 mM |
| Proliferation performance | + | ++ | ++ | ++ |

### Differentiative ability Test I

In a culture dish (60-mm dish) coated with fibronectin at 2.5 µg/cm², the same commercially available stem cells as those used in Proliferation Performance Test I were seeded at 5 × 10³ cells/cm² with any one of the media for stem cells of Example 8, Example 13, and Comparative Example 1 in a scale of 3 mL, and the stem cells were statically cultured under conditions of 37°C and 5% CO₂. When the cells in the culture dish were grown to subconfluency (confluency of about 80 to 90%), the same operation as for the cell passage described above was performed. The stem cells were removed from the culture dish, and 1 × 10⁵ cells were recovered in each of 1.5-mL tubes. The recovered stem cells were washed with 1 × PBS, and the antibody shown below was added to cause a reaction at 4°C for 30 minutes or more. After that, the stem cells were again washed with 1 × PBS, and analyzed by flow cytometry using a flow cytometer (available from Becton Dickinson, model: FACS Calibur) to check the expression of the antigen on the surface of the recovered stem cells. The flow cytometry was performed in accordance with the manual of the flow cytometer.

The following antibodies were used in Differentiative ability Test I.
- FITC Mouse IgG1, κ isotype Ctrl (available from BioLegend, Inc., product number: 400108)
- PE Mouse IgG1, κ isotype Ctrl (available from BioLegend, Inc., product number: 400112)
- PerCP/Cyanine5.5 Mouse IgG1, κ isotype Ctrl (available from BioLegend, Inc., product number: 400150)
- FITC anti-human CD90 (Thy1) (available from BioLegend, Inc., product number: 328108)
- PE anti-human CD105 (available from BioLegend, Inc., product number: 323206)
- PerCP/Cyanine5.5 anti-human CD73 (Ecto-5'-nucleotidase) (available from BioLegend, Inc., product number: 344014)
- FITC anti-human CD235a (available from BioLegend, Inc., product number: 349105)
- PE anti-human CD45 (available from BioLegend, Inc., product number: 304008)
- PerCP/Cyanine5.5 anti-human CD31 (available from BioLegend, Inc., product number: 303131)

Among the proteins to which the above-described antibodies bind, CD90, CD105, and CD73, which are expressed on the cell surface, are marker proteins of mesenchymal stem cells, and CD235a, CD45, and CD31 are negative marker proteins of mesenchymal stem cells.

### Differentiative ability Test II

The same operation for cell passage as in Proliferation Performance Test II was performed for the stem cells four times using any one of the media for stem cells of Example 8, Example 13, and Comparative Example 1, and the resulting stem cells were seeded in a 48-well plate for culturing cells at 2 × 10⁴ cells/well, and cultured until the cells were grown to a confluency of about 100%. Next, the medium was exchanged with a medium for inducing differentiation into osteoblast, chondrocyte, or adipocyte, and induction of differentiation was performed for about three weeks. The stem cells under induction of differentiation were statically cultured under conditions of 37°C and 5% CO₂.

The following media for inducing differentiation were used in Differentiative ability Test II.

### • Medium for inducing differentiation into osteoblast

This medium was obtained by adding, to an α-MEM medium, fetal bovine serum to a final concentration of 10%, dexamethasone to a final concentration of 10 nM, β-glycerophosphate to a final concentration of 10 mM, ascorbic acid to a final concentration of 50 µg/mL, and penicillin-streptomycin to a final concentration of 0.1%.

### • Medium for inducing differentiation into chondrocyte

This medium was obtained by adding, to an α-MEM medium, fetal bovine serum to a final concentration of 10%, dexamethasone to a final concentration of 100 nM, ascorbic acid to a final concentration of 25 µg/mL, and pyruvic acid to a final concentration of 1 mM.

### • Medium for inducing differentiation into adipocyte

This medium was obtained by adding, to a DMEM-High Glucose medium, fetal bovine serum to a final concentration of 10%, IBMX to a final concentration of 0.5 mM, dexamethasone to a final concentration of 1 µM, indomethacin to a final concentration of 200 µM, and penicillin-streptomycin to a final concentration of 0.1%.

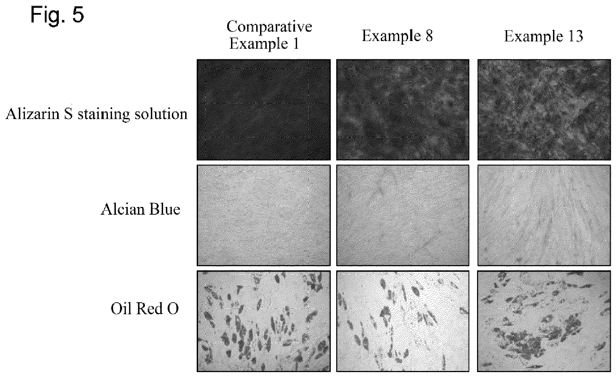

After inducing differentiation with the differentiation media, the supernatant was removed from the 48-well plate for culturing cells. Then, 0.99% PFA was added thereto, and the resultant was left to stand for 30 minutes to fix the cells. After that, PFA was removed, followed by washing with water. A cell staining solution was added thereto, and the resultant was left to stand for 20 minutes to stain the cells. After the cell staining, the cells were washed with Milli-Q water three times or more, and the state of the stained cells was observed using a microscope. The observation image is shown in Fig. 5.

In Differentiative ability Test II, the following staining solutions were used for staining the cells.

### • Alizarin S staining solution (available from Merck Millipore, product number: TMS_008_C)

The alizarin S staining solution contains a dye that is to bind to calcium, and thus the degree of differentiation into osteoblasts can be evaluated on the basis of the degree of staining with the staining solution.

### • Alcian Blue (available from Nacalai Tesque, Inc., product number: 37154-44)

The alcian blue can stain the extracellular matrix of chondrocytes, such as aggrecan or glycosaminoglycan, and thus the degree of differentiation into chondrocytes can be evaluated on the basis of the degree of staining with the alcian blue.

### • Oil Red O (available from Nacalai Tesque, Inc., product number: 25633-92)

The oil red O can stain lipid droplets, and thus the degree of differentiation into adipocytes can be evaluated on the basis of the degree of staining with oil red O.

As is clear from the results obtained in Proliferation Performance Test I, higher levels of proliferation performance for stem cells were observed when the media for stem cells of Examples 1 to 7, in which CMC was added, and the medium for stem cells of Example 11, in which PVP was added, were used, as compared with that observed when the medium of Comparative Example 1 was used (see Figs. 1 and 2). Such proliferation performance was observed even when the stem cells were subjected to cell passage culture in Proliferation Performance Test II (see Tables 2 and 3). Also, the population doubling level of stem cells was larger when the media for stem cells of Examples 1 to 7, in which CMC was added, and the medium for stem cells of Example 11, in which PVP was added, were used, as compared with that found when the medium of Comparative Example 1, in which none of CMC and PVP was added, was used (see Figs. 3(a) and 3(b)).

As is clear from the results obtained in Differentiative ability Test I, the stem cells cultured in the medium for stem cells of Example 8, in which CMC was added, and the stem cells cultured in the medium for stem cells of Example 13, in which PVP was added, favorably maintained their undifferentiated state, as with the stem cells cultured in the medium of Comparative Example 1, in which neither CMC nor PVP was added (see Fig. 4).

Also, in Differentiative ability Test II, the stem cells cultured in the medium for stem cells of Example 8, in which CMC was added, and the stem cells cultured in the medium for stem cells of Example 13, in which PVP was added, successfully differentiated into osteoblasts, chondrocytes, or adipocytes, as with the stem cells cultured in the medium of Comparative Example 1, in which neither CMC nor PVP was added (see Fig. 5). In other words, it was demonstrated that CMC or PVP causes no change in the differentiative ability of stem cells.

From the results obtained in Proliferation Performance Tests I and II and Differentiative ability Tests I and II, it was found that the medium for stem cells of the present invention, in which CMC or PVP is added, does not negatively affect the maintenance and proliferation of stem cells, and can exhibit improved proliferation performance while maintaining the differentiative ability of stem cells. Thus, it is possible to more stably and more efficiently culture stem cells.

### Industrial Applicability

The medium for stem cells according to the present invention, which contains at least one water-soluble polymer selected from carboxymethyl cellulose and polyvinylpyrrolidone in a basal medium necessary for culturing stem cells, exhibits a favorable proliferation performance while maintaining the differentiative ability of stem cells. Accordingly, use of the medium for stem cells enables more stable and more efficient culture of stem cells.

## Claims

1. A medium for stem cells, comprising at least one of carboxymethyl cellulose and polyvinylpyrrolidone as a water-soluble polymer.

2. The medium for stem cells according to claim 1,
wherein a content of carboxymethyl cellulose in the medium is such that a final concentration thereof is 0.001 µg/mL to 1 mg/mL.

3. The medium for stem cells according to claim 1,
wherein a content of polyvinylpyrrolidone in the medium is such that a final concentration thereof is 0.05 µg/mL to 2 mg/mL.

4. The medium for stem cells according to any one of claims 1 to 3, wherein a content of recombinant albumin in the medium is such that a final concentration thereof is 0.05 to 1 mg/mL.

5. The medium for stem cells according to any one of claims 1 to 4, further comprising β-nicotinamide mononucleotide.

6. A method for culturing stem cells, comprising culturing stem cells in the medium for stem cells according to any one of claims 1 to 5.
